Europäisches Patentamt

European Patent Office

Office européen des brevets

(19)

(11) Veröffentlichungsnummer : **0 381 988 B1**

(12) # EUROPÄISCHE PATENTSCHRIFT

(45) Veröffentlichungstag der Patentschrift :
**08.04.92 Patentblatt 92/15**

(51) Int. Cl.⁵ : **B01J 31/40,** C07C 51/42,
C07C 53/08, C07C 51/573,
C07C 53/12

(21) Anmeldenummer : **90101306.0**

(22) Anmeldetag : **23.01.90**

(54) **Verfahren zur Entfernung metallischer Korrosionsprodukte aus einer bei der Carbonylierung von Methanol und/oder Methylacetat und/oder Dimethylether anfallenden, verunreinigten Katalysatorlösung.**

(30) Priorität : **10.02.89 DE 3903909**

(43) Veröffentlichungstag der Anmeldung :
**16.08.90 Patentblatt 90/33**

(45) Bekanntmachung des Hinweises auf die
Patenterteilung :
**08.04.92 Patentblatt 92/15**

(84) Benannte Vertragsstaaten :
**AT BE CH DE ES FR GB IT LI NL**

(56) Entgegenhaltungen :
**EP-A- 0 032 525
EP-A- 0 073 342
EP-A- 0 156 253
EP-A- 0 240 703**

(73) Patentinhaber : **HOECHST
AKTIENGESELLSCHAFT
Brüningstrasse 50
W-6230 Frankfurt/Main 80 (DE)**

(72) Erfinder : **Erpenbach, Heinz, Dr.
Oberbuschweg 22
W-5000 Köln (DE)**
Erfinder : **Lork, Winfried
Siegfried-von-Westerburg-Strasse 14
W-5042 Erftstadt (DE)**
Erfinder : **Seidel, Andreas, Dr.
Luxemburger Strasse 469
W-5000 Köln (DE)**
Erfinder : **Prinz, Peter
Von-Geyr-Ring 104
W-5030 Hürth (DE)**

EP 0 381 988 B1

## Beschreibung

Die Erfindung betrifft ein Verfahren zur Entfernung metallischer Korrosionsprodukte umfassend wasserlöslische Verbindungen der Metalle Fe, Cr, Mo und Ni aus einer bei der Carbonylierung von Methanol und/oder Methylacetat und/oder Dimethylether anfallenden, verunreinigten Katalysatorlösung, welche 0,5-15 Masse% Carbonyl-Komplexe von Edelmetallen der Gruppe VIII des Periodensystems der Elemente, 2o-7o Masse% quaternäre Organophosphorverbindungen als organische Promotoren, 2-15 Masse% metallische Korrosionsprodukte, umfassend wasserlöslische Verbindugen der Metalle Fe, Cr, Mo und Ni sowie 13-75 Masse% Essigsäure, Essigsäureanhydrid, Ethylidendiacetat und ggf. undestillierbare organische Verbindungen enthält.

Die bei der Carbonylierung von Methanol und/oder Methylacetat und/oder Dimethylether im Kreislauf geführte Katalysatorlösung wird sowohl durch im Prozeß gebildete undestillierbare organische Verbindungen, welche als Harze oder Teere bezeichnet werden, als auch durch Anreicherung von metallischen Korrosionsprodukten wie Eisen, Nickel, Chrom und Molybdän verunreinigt. Ein Konzentrationsanstieg der metallischen Korrosionsprodukte beeinflußt neben der Katalysatorkreislaufführung den Carbonylierungsablauf durch Bildung unerwünschter Nebenprodukte, z.B. Aceton, Kohlendioxid und Methan.

Während die Reinigung der bei Carbonylierungsverfahren eingesetzten Katalysatorsysteme von undestillierbaren organischen Verbindungen schon häufiger beschrieben wurde, ist über die Rückgewinnung von Rhodium bzw. Edelmetallkomplexen aus mit metallischen Korrosionsprodukten verunreinigten Katalysatorlösungen bisher wenig bekannt.

So wird in der DE-A- 23 58 41o (= US-A-3 887 489) die rhodiumhaltige Katalysatorlösung, die durch Mischen einer Rhodiumkomponente und einer Iodkomponente, gewöhnlich Iodwasserstoff oder Alkyliodid, in Gegenwart von CO erhalten wurde, von den Korrosionsprodukten Fe, Ni, Cr und Mo dadurch befreit, daß die Rhodiumkomponente durch Erhitzen unter Rühren und ggf. unter Zusatz eines Alkanols mit 1-5 C-Atomen bei einer Temperatur von 1oo-19o°C ausgefällt und von den in Lösung verbleibenden Korrosionsprodukten abgetrennt wird.

Die gleiche, zuvor beschriebene Katalysatorlösung, die anstelle des Rhodiums auch Iridium enthalten kann, wird gemäß DE-A- 26 59 173 dadurch von metallischen Korrosionsprodukten gereinigt, daß sie mit stark sauren Kationenaustauscherharzen bei Temperaturen von 0-12o°C behandelt wird. Die Harze liegen in der H-Form vor und binden die Korrosionsprodukte, während der Rh-haltige Katalysatorkomplex in Lösung bleibt.

Die EP-A-0 2 65 140 beansprucht ein Verfahren, bei dem die verunreinigte Katalysatorlösung, die neben einer Rhodium- auch eine Lithiumkomponente enthält, dadurch von metallischen Korrosionsprodukten gereinigt wird, daß sie mit einem Kationenaustauscher in der Li-Form behandelt wird. Durch die Anwendung des Kationenaustauschers in der Li-Form wird eine Verminderung des Li-Gehaltes in der Lösung verhindert, da am Ionenaustauscher hauptsächlich die Korrosionsprodukte gebunden werden.

Für die Reinigung und Rückgewinnung des Edelmetallcarbonyl-Komplexes und der organischen Promotoren aus der bei der Carbonylierung von Methanol und/oder Methylacetat und/oder Dimethylether verunreinigten Katalysatorlösung sind die beschriebenen Methoden zur Entfernung von metallischen Korrosionsprodukten nicht anwendbar.

Bei den aus der Literatur bekannten Methoden stammen die zu reinigenden Katalysatorlösungen insbesondere aus Carbonylierungsverfahren zur alleinigen Herstellung von Essigsäure aus Methanol und CO. Diese Verfahren verlangen zu einer optimalen Reaktionsführung im Reaktionsgemisch Wassergehalte bis zu ca. 15 Masse%; organische undestillierbare Verbindungen fallen hierbei nicht an. Auch erfordern die angewendeten Katalysatorsysteme nicht unbedingt einen organischen Promotor, insbesondere nicht in einer so hohen Konzentration wie bei der gleichzeitigen Herstellung von Essigsäure und Essigsäureanhydrid oder auch von Essigsäureanhydrid allein. Die bei der Herstellung von Essigsäure/Essigsäureanhydrid eingesetzten Katalysatoren und die ggf. im Prozeß anfallenden undestillierbaren organischen Verbindungen sind wasserunlöslich und würden deshalb in den genannten Verfahren ausfallen. Die Gewinnung von Essigsäureanhydrid ist im wäßrigen System unter den Bedingungen einer Carbonylierung nicht möglich.

Der Einsatz von Ionenaustauschern zur Reinigung einer unter wasserfreien Bedingungen eingesetzten Katalysatorlösung führt im Falle vorliegender Erfindung neben einer partiellen Adsorption von undestillierbaren organischen Verbindungen am Austauscherharz auch zu einem Austausch des rhodiumhaltigen Komplex-Ions aus der Katalysatorlösung. Dadurch wird der Austauscher schneller inaktiviert und es kommt zu Verlusten des kostspieligen Rhodiumkatalysators. Der hohe Promotorsalzanteil in der Katalysatorlösung zeigt im übrigen, daß eine Entfernung der Korrosionsprodukte ohne Verluste nicht nur an Rhodium, sondern auch an Promotorsalz notwendig ist.

Die vorliegende Erfindung vermeidet die in dem angeführten Stand der Technik aufgezeigten Nachteile und beschreibt ein Verfahren, das es auf einfache Weise mittels extraktiver Methoden ermöglicht, die bei der Carbonylierung von Methanol und/oder Methylacetat und/oder Dimethylether eingesetzte und im Laufe des

EP 0 381 988 B1

Prozesses mit metallischen Korrosionsprodukten verunreinigte Katalysatorlösung so aufzuarbeiten, daß unter Abtrennung der metallischen Korrosionsprodukte der Edelmetallkomplex verlustfrei zurückgewonnen und die organischen Promotoren dem Katalysator-Kreislauf wieder zugeführt werden können.

Im einzelnen ist die Erfindung dadurch gekennzeichnet, daß man die Katalysatorlösung mit Wasser behandelt, wobei der Edelmetall-Carbonyl-Komplex und ggf. die undestillierbaren organischen Verbindungen ausgefällt werden, während der organische Promotor sowie die metallischen Korrosionsprodukte gelöst bleiben, daß man die wäßrige Phase von dem ausgefällten Edelmetall-Carbonyl-Komplex und ggf. den undestillierbaren organischen Verbindungen abtrennt und den organischen Promotor aus der wäßrigen Phase mit $C_4$-$C_8$-Alkanolen extrahiert, durch Abdampfen des Extraktionsmittels den Promotor zurückgewinnt, den Promotor mit dem ausgefällten Edelmetall-Carbonyl-Komplex und ggf. den undestillierbaren organischen Verbindungen wieder vereinigt und nach Zusatz von Essigsäure und/oder Essigsäureanhydrid als Katalysatorlösung in den Prozeß zurückführt, und daß man die wäßrige Phase mit den metallischen Korrosionsprodukten ausschleust.

Das Verfahren der Erfindung kann weiterhin bevorzugt und wahlweise dadurch gekennzeichnet sein, daß man

a) die Behandlung der verunreinigten Katalysatorlösung mit Wasser bei 1o-1oo°C vornimmt;
b) je Gewichtsteil verunreinigter Katalysatorlösung 3-1oo Gewichtsteile Wasser einsetzt.

Die mit metallischen Korrosionsprodukten verunreinigte Katalysatorlösung entstammt einer aus dem Carbonylierungsreaktor abfließenden Reaktionsmischung, die destillativ in die gewünschten Endprodukte Essigsäure und Essigsäureanhydrid sowie nicht umgesetzte, im Kreislauf geführte Ausgangsstoffe einerseits und die als Sumpfprodukt anfallende Katalysatorlösung andererseits aufgetrennt wird. Ein Teilstrom dieser mit der Zeit verunreinigten Katalysatorlösung wird aus dem Katalysator-Kreislauf entnommen und erfindungsgemäß von den metallischen Korrosionsprodukten befreit.

Die verunreinigten Katalysatorlösungen enthalten als Edelmetalle im allgemeinen Rhodium, Iridium, Palladium und/oder Ruthenium, die als Carbonylkomplexe wie z.B. $[CH_3P(C_4H_9)_3]_2$ $[Rh(CO)I_5]$, $[CH_3P(C_4H_9)_3][Rh(CO)I_4]$ oder $[CH_3P(C_4H_9)_3]$ $[Rh(CO)_2I_2]$ vorliegen. Die Katalysatorlösungen enthalten ferner als organische Promotoren bevorzugt einen oder mehrere der folgenden Organophosphorverbindungen:

Tetrabutylphosphoniumiodid, Tri-n-butyl-methyl-phosphoniumiodid, Trioctyl-methyl-phosphoniumiodid, Trilauryl-methyl-phosphoniumiodid und/oder Triphenylmethyl-phosphoniumiodid.

Der abgezogene Teilstrom wird zweckmäßig unter Rühren in z.B. auf 5o°C aufgeheiztes Wasser eindosiert. Während der Edelmetallcarbonylkomplex und ggf. die undestillierbaren organischen Verbindungen ausfallen, wird der organische Promotor mit den metallischen Korrosionsprodukten in der Wasserphase gelöst. Die wäßrige Phase wird vom ausgefällten Edelmetallkomplex abgetrennt und zur Rückgewinnung des Promotorsalzes einer extraktiven Behandlung mit z.B. n- oder i-Butanol oder Isoamylalkohol unterzogen. Nach Abdampfen des Extraktionsmittels verbleibt der organische Promotor, der dann mit dem ausgefällten und von metallischen Korrosionsprodukten befreiten Katalysatorkomplex und ggf. undestillierbaren organischen Verbindungen wieder vereinigt und unter Zusatz von Essigsäure und/oder Essigsäureanhydrid zum erneuten Einsatz als Katalysatorlösung in die Carbonylierungszone zurückgeführt wird. Die metallischen Korrosionsprodukte werden mit der wäßrigen Phase ausgeschleust.

## Beispiel 1

Zur Entfernung der metallischen Korrosionsprodukte werden dem Katalysatorkreislauf der Methanol-/Methylacetatcarbonylierung 1ooo g Katalysatorlösung der Zusammensetzung 5,51 Masse% Rhodiumcarbonylkomplex $[CH_3P(C_4H_9)_3][Rh(CO)_2I_2]$ ($\hat{=}$ 55,1 g = 0,90 Masse% Rh) , 53,72 Masse% Methyl-tri-n-butylphosphoniumiodid, 1,5 Masse% Eisen, 0,28 Masse% Nickel, 0,24 Masse% Chrom, 0,03 Masse% Molybdän und 29,4 Masse% eines Gemisches aus Essigsäure, Essigsäureanhydrid und Ethylidendiacetat entnommen und unter Rühren innerhalb von 30 Minuten in 5ooo ml Wasser von 8o°C eingegeben, wobei der Rhodiumcarbonylkomplex ausfällt. Danach wird der Rührer abgestellt. Nach einer Absetzzeit von 1,5 Stunden hat sich der Rhodiumcarbonylkomplex am Boden des Gefäßes niedergeschlagen, so daß die wäßrige Phase ohne Filtration durch Abhebern abgetrennt werden kann. Die Wasserphase wird anschließend zur Rückgewinnung des Promotors dreimal mit je 1ooo ml n-Butanol ausgeschüttelt. Aus der n-Butanolphase werden nach Abdampfen des n-Butanols 522,7 g Methyl-tri-n-butyl-phosphoniumiodid, entsprechend einer Ausbeute von 97,3 %, zurückgewonnen.

Der im Rührgefäß niedergeschlagene Carbonylkomplex wird mit dem zurückgewonnenen Promotorsalz und 14,5 g frischem Salz vereinigt und nach Auffüllen mit einem Essigsäure- Essigsäureanhydrid-Ethylidendiacetat-Gemisch auf 1ooo g als regenerierte Katalysatorlösung dem Katalysatorkreislauf wieder zugesetzt.

Mit der vom Promotorsalz befreiten Wasserphase werden nach Abstreifen des n-Butanols 14,6o g Eisen, 2,791 g Nickel, 2,395 g Chrom und 0,29 g Molybdän in Form der Iodide aus dem Prozeß ausgeschleust.

3

Die in der zurückgeführten Katalysatorlösung analytisch ermittelten Gehalte an 0,90 Masse% Rh, 0,06 Masse% Eisen, 0,004 Masse% Nickel, 0,005 Masse% Chrom und < 0,001 Masse% Molybdän zeigen die Effektivität der Reinigungsmethode. Ohne Rhodiumverlust werden aus der dem Katalysatorkreislauf abgezogenen Lösung die Korrosionsprodukte Eisen, Nickel, Chrom und Molybdän zu 97,3 %, 99,7 %, 99,8 % und > 96,7 % entfernt.

Beispiel 2

Zur Entfernung der metallischen Korrosionsprodukte werden dem Katalysatorkreislauf der Methanol-/Methylacetatcarbonylierung 1ooo g Katalysatorlösung der Zusammensetzung 7,47 Masse% Rhodiumcarbonylkomplex $[CH_3P(C_4H_9)_3][Rh(CO)_2I_2]$ (= 74,7 g = 0,87 Masse% Rh), 55,45 Masse% Methyl-tri-n-butylphosphoniumiodid, 0,85 Masse% Eisen, 0,13 Masse% Nickel, 0,15 Masse% Chrom, 0,02 Masse% Molybdän und 3o,7 Masse% eines Gemisches aus Essigsäure, Essigsäureanhydrid und Ethylidendiacetat entnommen und unter Rühren innerhalb von 30 Minuten in 6ooo ml Wasser von 45°C eingegeben, wobei der Rhodiumcarbonylkomplex ausfällt. Danach wird der Rührer abgestellt. Nach einer Absetzzeit von 1,5 Stunden hat sich der Rhodiumcarbonylkomplex am Boden des Gefäßes niedergeschlagen, so daß die wäßrige Phase ohne Filtration durch Abhebern abgetrennt werden kann. Die Wasserphase wird anschließend zur Rückgewinnung des Promotors dreimal mit je 1ooo ml Isoamylalkohol ausgeschüttelt. Aus der Isoamylalkoholphase werden nach Abdampfen des Isoamylalkohols 544,0 g Methyltri-n-butyl-phosphoniumiodid, entsprechend einer Ausbeute von 98,1 %, zurückgewonnen.

Der im Rührgefäß niedergeschlagene Carbonylkomplex wird mit dem zurückgewonnenen Promotorsalz und 1o,5 g frischem Salz vereinigt und nach Auffüllen mit einem Essigsäure-Essigsäureanhydrid-Ethylidendiacetat-Gemisch auf 1ooo g als regenerierte Katalysatorlösung dem Katalysatorkreislauf wieder zugesetzt.

Mit der vom Promotorsalz befreiten Wasserphase werden nach Abstreifen des Isoamylalkohols 8,1o g Eisen, 1,25 g Nickel 1,47 g Chrom und 0,19 g Molybdän in Form der Iodide aus dem Prozeß ausgeschleust.

Die in der zurückgeführten Katalysatorlösung analytisch ermittelten Gehalte an 0,87 Masse% Rh, 0,04 Masse% Eisen, 0,005 Masse% Nickel, 0,003 Masse% Chrom und < 0,001 Masse% Molybdän zeigen die Effektivität der Reinigungsmethode. Ohne Rhodiumverlust werden aus der dem Katalysatorkreislauf abgezogenen Lösung die Korrosionsprodukte Eisen, Nickel, Chrom und Molybdän zu 95,3 %, 96,2 %, 98,0 % und > 95,0 % entfernt.

Beispiel 3

Zur Entfernung der metallischen Korrosionsprodukte werden dem Katalysatorkreislauf der Dimethylethercarbonylierung 1000 g Katalysatorlösung der Zusammensetzung 10,96 M.-% Rhodiumcarbonylkomplex $[CH_3P(C_4H_9)_3]_2[Rh(CO)I_5]$ (= 109,6 g = 0,94 M.-% Rh) 51,99 M.-% Methyl-tri-n-butylphosphoniumiodid, 0,62 M.-% Eisen, 0,11 M.-% Nickel, 0,14 M.-% Chrom, 0,02 M.-% Molybdän und 32,1 M.-% eines Gemisches aus Essigsäure, Essigsäureanhydrid und Ethylidendiacetat entnommen und unter Rühren innerhalb von 30 Minuten in 4500 ml Wasser von 60°C eingegeben, wobei der Rhodiumcarbonylkomplex ausfällt. Danach wird der Rührer abgestellt. Nach einer Absetzzeit von 1,5 Stunden hat sich der Rhodiumcarbonylkomplex am Boden des Gefäßes niedergeschlagen, so daß die wäßrige Phase ohne Filtration durch Abhebern abgetrennt werden kann. Die Wasserphase wird anschließend zur Rückgewinnung des Promotors dreimal mit je 1000 ml n-Octanol ausgeschüttelt. Aus der n-Octanolphase werden nach Abdampfen des n-Octanols 508,5 g Methyl-tri-n-butylphosphoniumiodid, entsprechend einer Ausbeute von 97,8 %, zurückgewonnen.

Der im Rührgefäß niedergeschlagene Carbonylkomplex wird mit dem zurückgewonnenen Promotorsalz und 11,4 g frischem Salz vereinigt und nach Auffüllen mit einem Essigsäure-Essigsäureanhydrid-Ethylidendiacetat-Gemisch auf 1000 g als regenerierte Katalysator-Lösung dem Katalysatorkreislauf wieder zugesetzt.

Mit der vom Promotorsalz befreiten Wasserphase werden nach Abstreifen des n-Octanols 6,08 g Eisen, 1,07 g Nickel, 1,34 g Chrom und 0,19 g Molybdän in Form der Iodide aus dem Prozeß ausgeschleust.

Die in der zurückgeführten Katalysatorlösung analytisch ermittelten Gehalte an 0,94 M.-% Rh, 0,012 M.-% Eisen, 0,0025 M.-% Nickel, 0,006 M.-% Chrom und < 0,001 M.-% Molybdän zeigen die Effektivität der Reinigungsmethode. Ohne Rhodiumverlust werden aus der dem Katalysatorkreislauf abgezogenen Lösung die Korrosionsprodukte Eisen, Nickel, Chrom und Molybdän zu 98,1, 97,7, 95,7 und > 95,0 % entfernt.

**Patentansprüche**

1. Verfahren zur Entfernung metallischer Korrosionsprodukte, umfassend wasserlöslische Vervindungen der Metalle Fe, Cr, Mo und Ni aus einer bei der Carbonylierung von Methanol und/oder Methylacetat und/oder Dimethylether anfallenden, verunreinigten Katalysatorlösung, welche 0,5-15 Masse% Carbonyl-Komplexe von Edelmetallen der Gruppe VIII des Periodensystems der Elemente, 2o-7o Masse% quaternäre Organophosphorverbindungen als organische Promotoren, 2-15 Masse% metallische Korrosionsprodukte sowie 13-75 Masse% Essigsäure, Essigsäureanhydrid, Ethylidendiacetat und ggf. undestillierbare organische Verbindungen enthält, dadurch gekennzeichnet, daß man die Katalysatorlösung mit Wasser behandelt, wobei der Edelmetall-Carbonyl-Komplex und ggf. die undestillierbaren organischen Verbindungen ausgefällt werden, während der organische Promotor sowie die metallischen Korrosionsprodukte gelöst bleiben; daß man die wäßrige Phase von dem ausgefällten Edelmetall-Carbonyl-Komplex und ggf. den undestillierbaren organischen Verbindungen abtrennt und den organischen Promotor aus der wäßrigen Phase mit $C_4$-$C_8$-Alkanolen extrahiert, durch Abdampfen des Extraktionsmittels den Promotor zurückgewinnt, den Promotor mit dem ausgefällten Edelmetall-Carbonyl-Komplex und ggf. den undestillierbaren organischen Verbindungen wieder vereinigt und nach Zusatz von Essigsäure und/oder Essigsäureanhydrid als Katalysatorlösung in den Prozeß zurückführt; und daß man die wäßrige Phase mit den metallischen Korrosionsprodukten ausschleust.

2. Verfahren nach Anspruch 1, dadurch gekennzeichnet, daß man die Behandlung der verunreinigten Katalysatorlösung mit Wasser bei 1o-1oo°C vornimmt.

3. Verfahren nach Anspruch 1 oder 2, dadurch gekennzeichnet, daß man je Gewichtsteil verunreinigter Katalysatorlösung 3-1oo Gewichtsteile Wasser einsetzt.

## Claims

1. A process for the removal of metallic corrosion products, comprising water-soluble compounds of the metals Fe, Cr, Mo and Ni, from a contaminated catalyst solution which is obtained during carbonylation of methanol and/or methyl acetate and/or dimethyl ether and contains 0.5-15 % by weight of carbonyl complexes of noble metals of group VIII of the periodic table of the elements, 20-70 % by weight of quaternary organophosphorus compounds as organic promoters, 2-15 % by weight of metallic corrosion products and 13-75 % by weight of acetic acid, acetic anhydride, ethylidene diacetate and if appropriate non-distillable organic compounds, which comprises treating the catalyst solution with water, the noble metal carbonyl complex and if appropriate the non-distillable organic compounds being precipitated while the organic promoter and the metallic corrosion products remain dissolved; separating off the aqueous phase from the precipitated noble metal carbonyl complex and if appropriate from the non-distillable organic compounds and extracting the organic promoter from the aqueous phase using $C_4$-$C_8$-alkanols, recovering the promoter by evaporation of the extraction agent, combining the promoter again with the precipitated noble metal carbonyl complex and if appropriate the non-distillable organic compounds and, after addition of acetic acid and/or acetic anhydride, recycling the mixture to the process as the catalyst solution; and draining off the aqueous phase containing the metallic corrosion products.

2. The process as claimed in claim 1, wherein the treatment of the contaminated catalyst solution with water is carried out at 10-100°C.

3. The process as claimed in claim 1 or 2, wherein 3-100 parts by weight of water are employed per part by weight of contaminated catalyst solution.

## Revendications

1. Procédé pour la séparation de produits de corrosion métalliques, comprenant des composés solubles dans l'eau des métaux Fe, Cr, Mo et Ni, d'une solution de catalyseur contaminée se formant dans la carbonylation du méthanol et/ou de l'acétate de méthyle et/ou de l'oxyde de diméthyle, qui contient 0,5-15 % en masse de complexes carbonyles de métaux nobles du groupe VIII de la Classification Périodique des Eléments, 20-70 % en masse de composés organophosphorés quaternaires comme promoteurs organiques, 2-15 % en masse de produits de corrosion métalliques ainsi que 13-75 % en masse d'acide acétique, d'anhydride acétique, de diacétate d'éthylidène et éventuellement de composés organiques non distillables; caractérisé en ce que l'on traite la solution de catalyseur par l'eau, ce qui précipite le complexe de métal noble-carbonyle et éventuellement les composés organiques non distillables, tandis que le promoteur organique ainsi que les produits de corrosion métalliques restent dissous ; en ce que l'on sépare la phase aqueuse du complexe de métal noble-carbonyle précipité et éventuellement des composés organiques non distillables et on extrait le promoteur organique de la phase aqueuse par des alcanols en $C_4$-$C_8$, on récupère le promoteur par évaporation de l'agent d'extraction, on réunit à nouveau le promoteur avec le complexe de métal noble-carbonyle précipité et éventuellement les composés organiques non distillables et on les renvoie dans le procédé comme solution de catalyseur après addition d'acide acétique et/ou d'anhydride acétique ; et en ce que l'on retire la phase aqueuse avec les produits de corrosion métalliques.

2. Procédé selon la revendication 1, caractérisé en ce que l'on effectue le traitement par l'eau de la solution de catalyseur contaminée à 10-100°C.

3. Procédé selon la revendication 1 ou 2, caractérisé en ce que l'on utilise 3-100 parties en poids d'eau par partie en poids de solution de catalyseur contaminée.